# EUROPEAN PATENT APPLICATION

(11) **EP 1 314 404 A2**
(43) Date of publication of application: **28.05.2003**
(21) Application number: 02022566.0
(22) Date of filing: 08.10.2002
(51) Int. Cl.: A61F 2/04, A61M 27/00

(54) **Endoscopic transduodenal biliary drainage system**

(30) Priority: 10.10.2001 JP 2001312248
(71) Applicant: Medico's Hirata Inc., Osaka-shi, Osaka 530-0003 (JP)
(72) Inventor: Kakutani, Hiroshi, Tokyo 176-0022 (JP); Hino, Shoryoku, Tokyo 152-0002 (JP)
(74) Representative: TER MEER STEINMEISTER & PARTNER GbR

(57) **Abstract**

The invention relate to a new procedure for physiologically draining bile into the duodenum, and provides a biliary drainage system for use in this procedure. The biliary drainage system comprises a puncture needle 2 having a sharp pointed portion 5 at a distal end thereof, an expandable metallic stent 1 provided on a distal end portion of the needle 2, and a delivery sheath 3 having the stent 1 enclosed therein in a contracted state. The system is useful for a surgical method comprising endoscopically puncturing the gallbladder 7 with the sharp pointed portion 5 of the needle 2 from the duodenum 6 while passing a highfrequency current through the needle 2, thereafter pulling the delivery sheath 3 to expand the stent 1 and cause both the duodenum 6 and the gallbladder 7 to retain the stent 1, and removing the needle 2.

## Description

### BACKGROUND OF THE INVENTION

### Field of the Invention

The present invention relates to endoscopic transduodenal biliary drainage for use in malignant diseases or acute inflammatory diseases the causes of which are currently unremovable clinically, such as obstructive jaundice and acute cholecystitis, and more particularly to drainage systems for use in this procedure.

### Description of the Prior Art

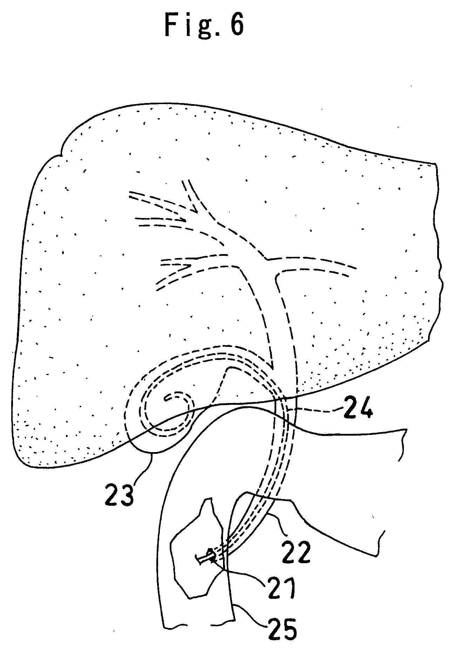

It is likely that elderly patients who are physically unable to withstand surgical operations will fail to drain bile into the duodenum due to the constriction of biliary tract to develop jaundice. Conventionally known for treating jaundice are a procedure comprising endoscopically inserting a guide wire into the biliary tract through the duodenal papilla, inserting a guide tube into the biliary tract with the wire serving as a guide, and subsequently fitting a drainage tube around the guide tube to insert the drainage tube into a constricted portion of the biliary tract for retention to provide a channel for draining bile, or a procedure comprising inserting a drainage tube 24 into the gallbladder 23 through the papilla 21 of the duodenum 25 and then through the bile ducts 22 and draining bile from the gallbladder 23 into the duodenum 25 through the tube 24 as shown in FIG. 6.

However, the patient can not be treated by such conventional procedures if the endoscope fails to reach the duodenal papilla or when the guide wire can not be inserted into the biliary tract.

The present invention relate to a new procedure for. physiologically draining bile into the duodenum free of the above problem, and an object of the invention is to provide a biliary drainage system for use in this procedure.

### SUMMARY OF THE INVENTION

The present invention has been accomplished to fulfill the above object.

The present invention provides as a first feature thereof an endoscopic transduodenal biliary drainage system comprising a puncture needle 2 having a sharp pointed portion 5 at a distal end thereof, an expandable metallic stent 1 provided on a distal end portion of the needle 2, and a delivery sheath 3 having the stent 1 enclosed therein in a contracted state, the biliary drainage system being adapted for use in a surgical method comprising enddscopically puncturing the gallbladder 7 with the sharp pointed portion 5 of the needle 2 from the duodenum 6 while passing a high-frequency current through the needle 2, thereafter pulling the delivery sheath 3 to expand the stent 1 and cause both the duodenum 6 and the gallbladder 7 to retain the stent 1, and removing the needle 2.

The first feature of the invention relates also to a surgical method of endoscopic transduodenal biliary .drainage which is to be practiced with use of the biliary drainage system described and which comprises endoscopically puncturing the gallbladder 7 with the sharp pointed portion 5 of the needle 2 from the duodenum 6 while passing a high-frequency current through the needle 2, thereafter pulling the delivery sheath 3 to expand the stent 1 and cause both the duodenum 6 and the gallbladder 7 to retain the stent 1, and removing the needle 2.

The present invention further provides as a second feature thereof an endoscopic transduodenal biliary drainage system comprising a puncture needle 2 having a sharp pointed portion 5 at a distal end thereof, a guiding catheter 10 having the needle 2 inserted therethrough, a tube stent 11 provided around a distal end portion of the needle 2, and a delivery sheath 3 provided around the catheter 10 on the proximal side of the stent 11, the biliary drainage system being adapted for use in a surgical method comprising endoscopically puncturing the gallbladder 7 with the sharp pointed portion 5 of the needle 2 from the duodenum 6 while passing a high-frequency current through the needle 2, and thereafter pulling the needle 2 and the catheter 10 to cause both the duodenum 6 and the gallbladder 7 to retain the stent 11.

The second feature of the invention relates also to a surgical method of endoscopic transduodenal biliary drainage which is to be practiced with use of the biliary drainage system described and which comprises endoscopically puncturing the gallbladder 7 with the sharp pointed portion 5 of the needle 2 from the duodenum 6 while passing a high-frequency current through the needle 2, and thereafter pulling the needle 2 and the catheter 10 to cause both the duodenum 6 and the gallbladder 7 to retain the stent 11.

### BRIEF DESCRIPTION OF THE DRAWINGS

FIG. 1 is a diagram showing a puncture needle 2 provided with a stent 1 as contracted and inserted through a delivery sheath 3 in Example 1;
FIG. 2 is a diagram showing the stent 1 as expanded in the form of an hourglass by pulling the delivery sheath 3;
FIG. 3 is a diagram showing the gallbladder 7 as punctured with/a conical sharp pointed portion 5 of the needle 2 through the duodenum (6), while a high-frequency current is being passed through the pointed portion 5;
FIG. 4 is a diagram showing the gallbladder 7 as joined directly to the duodenum 6 by the stent 1;
FIG. 5 is a diagram showing a system for use in Example 2 which system comprises a puncture needle 2 inserted through a guiding catheter 10, a tube stent 11 provided around a distal end portion of the needle, and a delivery sheath 3 provided around the catheter 10 on the proximal side of the stent 11; and
FIG. 6 is a diagram showing a conventional surgical method wherein a drainage tube 24 is inserted into the gallbladder 23 through the papilla 21 of the duodenum 25 and then through the bile ducts 22.

### DESCRIPTION OF THE PREFERRED EMBODIMENTS

The present invention uses as a first feature thereof an expandable metallic stent (hereinafter referred to briefly as "EMS") 1 which is made of metal netting. The EMS is in the form of an hourglass, hollow cylinder or the like when expanded, and is enclosed, as contracted by being radially folded to a compact form, in a distal end portion of a slender delivery sheath. The EMS is self-expandable, such that when withdrawn from the sheath, the stent restores itself to the original diameter owing to the resiliency or shape memory properties of its own. The stent is mainly prepared from a thin resilient metal wire of stainless steel (SUS316 or SUS304) or Nitinol (nickel-titanium alloy) and is in the form of a cylinder having a required diameter. The stent is in the form of a zigzag successively Z-shaped hollow cylinder or a hollow cylinder of netting so as to be expandable.

The EMS is preferably 25 to 40 mm in length. When expanded to the form of an hourglass, the EMS is preferably 6 to 20 mm in the outside diameter of larger end thereof and 3 to 6 mm in the outside diameter of constricted portion thereof.

Preferably, the EMS is a covered stent having a cover of thin film of silicone rubber, polyurethane, polyethylene, PTFE or the like formed over the outer surface of a stent frame to prevent ingress of living tissue or leakage of body fluid.

According to a second feature of the invention, use is made of a stent 11 which is a tube stent such as a pigtail tube stent having two poles or a pigtail tube stent having a single pole. Preferably, the tube stent is made from a synthetic resin such as Teflon or polyethylene. The tube stent is made straight by a puncture needle inserted therethrough, then brought to a predetermined position in this state and returned to the original pigtail form.

According to the second feature of the invention, use is made of a guiding catheter 10 which may be made from a synthetic resin such as Teflon or polyethylene. The catheter is preferably 3 to 4 mm in outside diameter.

The puncture needle 2 to be used according to the first and second features of the invention is made from an electrically conductive metal such as stainless steel, comprises a single wire or a strand of thin wires, and has a conical or pyramidal sharp pointed portion 5 at its distal end. The conical sharp pointed portion is preferably 2 to 20 mm in length and 0.8 to 3 mm in the diameter of enlarged rear end thereof.

The delivery sheath 3 for enclosing the stent therein is made from a synthetic resin such as Teflon or polyethylene and has an outside diameter approximately equal to that of the tube stent.

Next, a description will be given of endoscopic transduodenal biliary drainage .to be performed with use of the drainage system of the invention.

According to the first feature of the invention, an ultrasonic endoscope is inserted into the duodenum through the mouth, esophagus and stomach, and the gallbladder 7 is ultrasonically scanned through the duodenum 6 to recognize the position of the gallbladder and the distance to the gallbladder and to ascertain that the path to be used for puncturing is free from blood vessels. The delivery sheath 3 having enclosed therein the puncture needle 2 provided with the stent 1 is fitted into a forceps hole of the endoscope. The gallbladder is punctured through the duodenum with the needle 2 by inserting the needle 2 along the path checked ultrasonically, while passing a high-frequency current through the needle 2. After the needle 2 has reached the gallbladder, the position where the stent 1 is to be retained is determined. The delivery sheath 3 is then pulled to expand the stent 1 and cause the duodenum 6 and the gallbladder 7 to retain the stent 1. The puncture needle 2 is thereafter removed.

According to the second feature of the invention, the puncture needle 2 is inserted through the guiding catheter 10, the pigtail tube stent 11 having two poles is provided around a distal end portion of the needle, and the delivery sheath 3 is provided around the catheter 10 on the proximal side of the stent 11. Preferably, the tube stent is fixed to the delivery sheath as with a thread. The fixing thread or the like is made easily removable form the tube stent after the stent is retained in position. The pigtail stent 11 is made straight by the puncture needle 2 inserted therethrough, brought to the specified position in this state, thereafter returned to the pigtail form by withdrawing the needle 2 and the guiding catheter 10, and retained in position. The same procedure as is the case with the first feature of the invention is performed with the exception of using this system.

Next, Examples of the invention will be described with reference to the drawings concerned.

### Example 1

An ultrasonic endoscope (color Doppler ultrasonic endoscope of the convex scanning type), GF-UCT240-AL5 (product of Olympus Optical Co., Ltd.), or Pentax FG36UX (product of Asahi Optical Co., Ltd.) was inserted into the duodenum through the mouth, esophagus and stomach, and the gallbladder was ultrasonically scanned through the duodenum to recognize the position of the gallbladder and the distance to the gallbladder and to ascertain that the path to be used for puncturing was free from blood vessels.

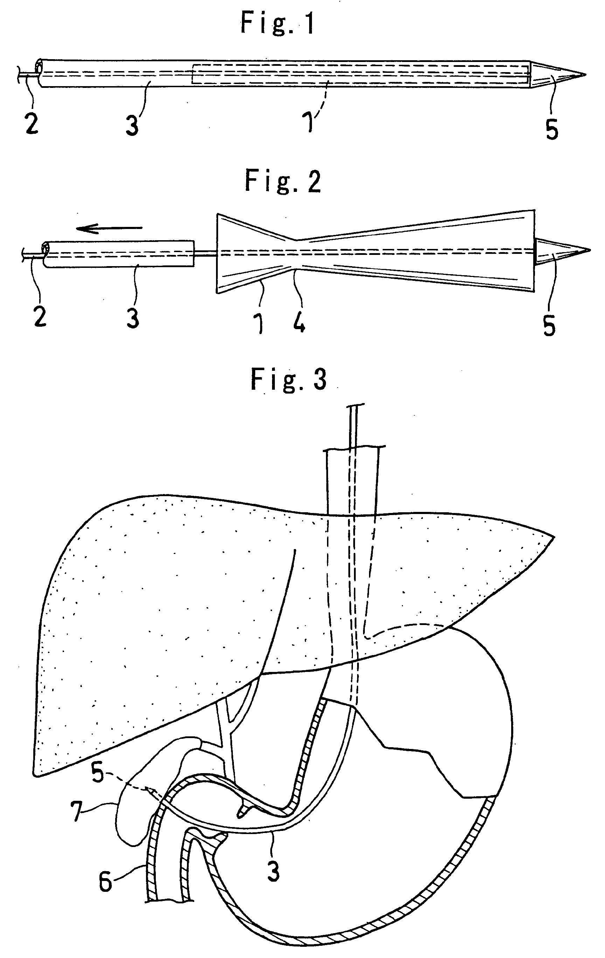

A delivery sheath 3 of Teflon 3.1 mm in outside diameter and having enclosed therein a puncture needle 2 provided with an expandable stent 1 of stainless steel netting as shown in FIG. 1 was fitted into a forceps hole of the endoscope. Although enclosed as contracted in the sheath 3, the stent 1 is in the form of an hourglass which is 4 cm in length, 10 mm in the outside diameter of opposite ends and 3.5 mm in the outside diameter of its constricted portion which is at a distance of 1 cm from one end when expanded. The stent 1 has a polyurethane cover provided over a zigzag successively Z-shaped stent frame. The stent 1 has a constricted portion 4 at a distance of 10 mm form one end thereof and is provided around the puncture needle 2, with the constricted portion 4 positioned closer to the proximal end, i.e., the rear end, of the needle 2. The puncture needle 2 comprises a strand of stainless steel wires, 0.9 mm in thickness, has at its distal end a conical sharp pointed portion 5, 7 mm in length and 3.1 mm in the diameter of enlarged rear end thereof.

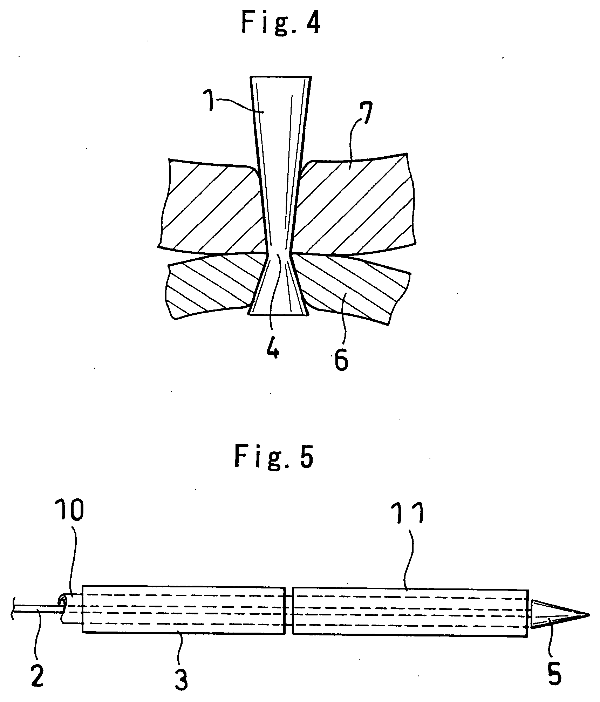

As shown in FIG. 3, the gallbladder 7 was punctured through the duodenum 6 with the sharp pointed portion 5 of the puncture needle 2 by inserting the needle 2 along a path checked ultrasonically, while passing a high-frequency current through the needle portion 5. After the sharp pointed portion 5 reached the gallbladder 7, images of the inside of the gallbladder were taken, and the position where the stent 1 was to be retained was determined. The delivery sheath 3 was then pulled to expand the stent 1 to the shape of an hourglass and retain the stent 1 in position. The puncture needle 2 was thereafter removed to join the gallbladder 7 directly to the duodenum 6 by the stent 1 as shown in FIG. 4, and bile was physiologically drained from the gallbladder 23 into the duodenum 25 through the stent.

### Example 2

In the drainage system shown in FIG. 5, the conical sharp pointed portion 5 of the puncture needle 2 has an enlarged rear end which is 2.3 mm in diameter. The needle 2 is inserted through a guiding catheter 10 having an outside diameter of 2.1 mm and provided on a distal end portion thereof with a Teflon pigtail stent 11 having two poles and an outside diameter of 3.1 mm. A delivery sheath 3, 3.1 mm in outside diameter, is provided around the catheter 10, as positioned close to the proximal end, i.e., rear end, of the stent 11. The stent 11 is made straight by the needle 2 inserted therethrough. The stent 11 in this state is placed into a predetermined position, and the needle 2 and the guiding catheter 10 are thereafter withdrawn, whereby the stent is returned to the form of a pigtail and retained in position. The same procedure as in Example 1 was performed with the exception of using the system described.

The biliary drainage systems of the present invention are usable by a facilitated procedure since there is no need to cause the drainage tube to reach the duodenal papilla as practiced in the conventional surgical method. The pain to be given to the patient is therefore mitigated.

Moreover, bile can be physiologically spontaneously discharged into the duodenum. The systems of the invention are advantageous in this respect.

## Claims

1. An endoscopic transduodenal biliary drainage system comprising a puncture needle (2) having a sharp pointed portion (5) at a distal end thereof, an expandable metallic stent (1) provided on a distal end portion of the needle (2), and a delivery sheath (3) having the stent (1) enclosed therein in a contracted state, the biliary drainage system being adapted for use in a surgical method comprising endoscopically puncturing the gallbladder (7) with the sharp pointed portion (5) of the needle (2) from the duodenum (6) while passing a high-frequency current through the needle (2), thereafter pulling the delivery sheath (3) to expand the stent (1) and cause both the duodenum (6) and the gallbladder (7) to retain the stent (1), and removing the needle (2).

2. An endoscopic transduodenal biliary drainage system comprising a puncture needle (2.) having a sharp pointed portion (5) at a distal end thereof, a guiding catheter (10) having the needle (2) inserted therethrough, a tube stent (11) provided around a distal end portion of the needle (2), and a delivery sheath (3) provided around the catheter (10) on the proximal side of the stent (11), the biliary drainage system being adapted for use in a surgical method comprising endoscopically puncturing the gallbladder (7) with the sharp pointed portion (5) of the needle (2) from the duodenum (6) while passing a high-frequency current through the needle (2), and thereafter pulling the needle (2) and the catheter (10) to cause both the duodenum (6).and the gallbladder (7) to retain the stent (11).

3. A surgical method of endoscopic transduodenal biliary drainage with use of a biliary drainage system comprising a puncture needle (2) having a sharp pointed portion (5) at a distal end thereof, an expandable metallic stent (1) provided on a distal end portion of the needle (2), and a delivery sheath (3) having the stent (1) enclosed therein in a contracted state, the surgical method comprising endoscopically puncturing the gallbladder (7) with the sharp pointed portion (5) of the needle (2) from the duodenum (6) while passing a high-frequency current through the needle (2), thereafter pulling the delivery sheath (3) to expand the stent (1) and cause both the duodenum (6) and the gallbladder (7) to retain the stent (1), and removing the needle (2).

4. A surgical method of endoscopic transduodenal biliary drainage with use of a biliary drainage system comprising a puncture needle (2) having a sharp pointed portion (5) at a distal end thereof, a guiding catheter (10) having the needle (2) inserted therethrough, a tube stent (11) provided around a distal end portion of the needle (2), and a delivery sheath.(3) provided around the catheter (10) on the proximal side of the stent (11), the surgical method comprising endoscopically puncturing the gallbladder (7) with the sharp pointed portion (5) of the needle (2) from the duodenum (6) while passing a high-frequency current through the needle (2), and thereafter pulling the needle (2) and the catheter (10) to cause both the duodenum (6) and the gallbladder (7) to retain the stent (11).
